(19) Europäisches Patentamt
European Patent Office
Office européen des brevets

(11) Publication number: 0 088 462
A2

(12) # EUROPEAN PATENT APPLICATION

(21) Application number: 83200240.6

(22) Date of filing: 16.02.83

(51) Int. Cl.³: A 61 K 45/06, A 61 K 31/66,
A 61 K 31/57
// (A61K31/66, 31/57)

(30) Priority: 01.03.82 US 353120

(43) Date of publication of application: 14.09.83
Bulletin 83/37

(84) Designated Contracting States: BE CH DE FR GB IT LI NL SE

(71) Applicant: THE PROCTER & GAMBLE COMPANY,
301 East Sixth Street, Cincinnati Ohio 45201 (US)

(72) Inventor: Baker, Bennie Lee, 1658 McIntyre Dr., Ann Arbor Michigan 48105 (US)
Inventor: Flora, Lawrence, 5850 Fairdale Dr., Fairfield Ohio 45014 (US)

(74) Representative: Suslic, Lydia et al, Procter & Gamble European Technical Center Temselaan 100, B-1820 Strombeek-Bever (BE)

(54) **Anti-inflammatory and rheumatic composition containing organophosphonate and steroid.**

(57) Compositions comprising steroid compounds and organophosphonates useful in the treatment of rheumatic conditions are disclosed. These compositions are effective in the treatment of inflammatory and rheumatic conditions. They utilize lower levels of the steroid than ordinarily required, while minimizing the side effects which frequently accompany steroid treatment. The method of treating arthritic conditions using these compositions is also disclosed.

EP 0 088 462 A2

0088462

# ANTI-INFLAMMATORY AND RHEUMATIC COMPOSITION CONTAINING ORGANOPHOSPHONATE AND STEROID

## TECHNICAL FIELD

The present invention relates to novel pharmaceutical compositions used in the treatment of arthritic and other inflammatory conditions.

## BACKGROUND OF THE INVENTION

Rheumatic diseases are conditions in which pain and stiffness are prominent in generalized or specific portions of the musculo-skeletal system. This can sometimes include connective tissue. They are generally idiopathic.

When the joints themselves are principally involved in the inflammation, the name most commonly associated with such inflammation is arthritis. Arthritis is one of the oldest known diseases, having plagued man's ancestors for some two million years. Yet the task of discovering a safe and effective treatment for it continues.

The economics of the disease render obvious the failure of present treatments. As a result of some 20 million individuals in the United States suffering from some form of rheumatic disease, a total of 27 million work days are lost each year. This 216 million hours away from the work place is second only to heart disease as a chronic cause of such losses.

Various steroids have been recognized as being effective in the treatment of rheumatoid diseases, such as arthritis. This has been especially true of glucocorticoids, a class of corticosteroids.

Glucocorticoids, originally named because of their characteristic effects on the intermediary-metabolism of glucose, were first recognized for their potent anti-inflammatory

properties in 1949. It was at this time that Hench reported the therapeutic benefits of cortisone in patients with rheumatoid arthritis. See Hench, et al. Mayo Clinic Procedure, 24:181 (1949). (He was subsequently awarded the Nobel prize in Medicine.) Because of the success reported by Hench and those who followed, the therapeutic use of glucocorticoids increased dramatically. They were used to treat a host of inflammatory diseases. It was quickly observed that the biochemical, pharmacologic and physiologic properties of glucocorticoids produce useful anti-inflammatory and immunosuppressive effects in most rheumatic diseases.

As with such discoveries, it also became readily apparent that the decision to use glucocorticoids also required constant, thorough, and detailed assessment of the subject's response to such a protocol; the numerous and serious side effects glucocorticoids were found to produce were sobering. Thus the decision to use glucocorticoids requires a clear definition of the benefits to be gained by such treatment, and identification of the risks the subject may encounter, so that an informed overall assessment may be made.

The risks attendent use of glucocorticoids include abnormal $Na^+$ reabsorption and $K^+$ excretion. Interference with these 2 important monovalent ions may result in hypokalemic alkalosis, edema, hypertension, and other abnormalities associated with electrolyte imbalances. Glucocorticoids can also suppress the natural healing process of injuries, especially those that cause a break in the integument. It is thought that glucocorticoids interfere with de novo synthesis of collagen by the fibroblasts resulting in scar tissue of reduced effectiveness which may easily dehisce as a result.

The immunosuppressive properties of glucosteroids (the very property which makes them valuable in treatment of immunologically mediated disease), can result in compromising the subject's ability to fight infection. They can also mask the symptoms of some infections, thus preventing or delaying diagnosis and treatment.

Other side-effects of glucocorticoid therapy include

causing abnormal function within the gastrointestinal, cardiovascular, endocrine and central nervous systems. These may occur in any subject receiving a supraphysiologic concentration of these hormones or their synthetic equivalent. In general, the frequency and severity of such side-effects are proportional to the dose given, and the duration of the treatment. When either the amount per dose, or the period of time over which therapy is given, is increased, an increase in side-effects can be anticipated. Such increases can be expected to be both in kind and degree.

Such side-effects are thought to be reversible by discontinuing or reducing glucocorticoid level, with the important exceptions of skeletal side-effects. The reversibility of such side-effects is often thwarted by increasing the duration of exposure to supra-physiologic levels of steroids. See McCarty, Arthritis and Allied Conditions, Lea & Febiger, 1979, p. 398, incorporated herein by reference.

The musculoskeletal side-effects, osteoporosis and aseptic necrosis of bone, are not totally reversible. Such risk of irreversible damage can result in glucocorticoid therapy being contraindicated in sound medical judgement even when the severity of the underlying disorder is great enough to otherwise easily justify initiating steroid therapy. In diseases that are not life-threatening, such as rheumatoid arthritis, more conservative therapy is frequently indicated. It is more sensible to use therapies without the attendant risk of permanent or even temporary but serious side-effects when the underlying disease or illness is not threatening survival. There clearly exists a need for a method of treatment and/or composition that would allow effective steroid therapy, but reduce or minimize steroid side effects.

When the above is translated into actual clinical practice, 2 rules become paramount when glucocorticosteroid therapy is to be administered in the treatment of any condition or episode:

(1) the period for administration must be as short as possible, and (2) the dose must be the smallest one that will achieve the desired effect.

When the therapy which calls for a single administration is indicated, the single administration can be viewed as innocuous in character. Short courses of systematic glucocorticoid thereapy of substantial doses may be proper even in conditions which are not life threatening, assuming the absence of specific contraindications. However, it is generally accepted that courses of long-term therapy at a high dosage should be reserved only for life-threatening disease. This rule is occasionally justifiably violated when the patient is threatened with significant and permanent disability. See Goodman and Gilman, The Pharmacological Basis of Therapeutics, MacMillan Publishing Co., Inc., 1975, 1497-1500, Chapter 70, incorporated herein by reference.

Though rheumatoid arthritis and other rheumatic conditions rarely threaten survival, they are frequently progressive, resulting in physical and economic disability despite intensive treatment with other protocols. This is generally when steroid therapy is indicated. However, as Goodman and Gilman indicate, the decision to institute glucocorticosteroid therapy must be undertaken in light of the fact that such therapy might well have to be continued for many years, perhaps for life.

The above reflects the existence of a need for a method of treatment and/or composition which would aid in the treatment of rheumatic conditions while (1) reducing the amount of steroid which must be administered (2) reducing the frequency/duration of such treatments and (3) reducing the side-effects that generally accompany the administration of steroid therapy, especially those that are partially or totally irreversible, such as the skeletal side-effects.

Organophosphate compounds are reported in the literature as being useful in the treatment and therapy of irregular or anomalous mobilization and deposition of calcium phosphate salts (bone mineral) in humans and other animals; use of the compounds in the treatment of arthritis is specifically disclosed. See, U.S. 3,683,080, Francis, granted August 8, 1972; U.S. 4,234,645, Gunther and Fleisch, issued November 18, 1980; and U.S. 4,216,212, Flora and Francis, issued August 5, 1980, all of which are incorporated herein by reference.

The article by Francis, Flora and King, entitled "The Effects of Disodium Ethane-1-Hydroxy-1,1-Diphosphonate on Adjuvant Induced Arthritis in Rats", appearing in Calc. Tiss. Res., 9:109-121 (1972) discloses the use of a diphosphonate material in rats and mentions the use of phosphonates to inhibit inflammatory erosion in rat cartilage.

One object of the present invention is to provide pharmaceutical compositions, comprising organophosphonates and steroids, which are effective in the treatment of inflammatory and rheumatic conditions, and episodes, especially rheumatic arthritis. It is also an object of this invention to provide pharmaceutical compositions, comprising organophosphonates and steroids, which are effective in the treatment of inflammatory and rheumatic conditions and episodes, especially rheumatic arthritis, while reducing and/or minimizing the side effects which normally accompany steroid treatment, especially those that are irreversible, such as the skeletal side effects.

It is a further object of this invention to provide a method of treatment for inflammatory and rheumatic conditions and episodes, especially rheumatic arthritis, using combinations of organophosphonates and steroids.

The present invention encompasses compositions and methods for treating arthritic and other rheumatic conditions in animal tissue, especially in humans. The invention provides effective drug combinations, compositions and therapy, and is based on the use of pharmacologically active and pharmaceutically acceptable organophosphonate compounds together with pharmacologically-active and pharmaceutically-acceptable steroid compounds useful in the treatment of such conditions.

The compositions of this invention comprise a safe and effective amount of an organophosphate compound, particularly a geminal diphosphonate such as dichloromethane diphosphonic acid, or ethane-1-hydroxy-1, 1-diphosphonic acid, together with a safe and effective amount of a pharmacologically-active and

pharmaceutically-acceptable steroid useful in the treatment of rheumatic conditions, such as prednisolone. The compounds act in concert to provide effective treatment of the rheumatic condition while minimizing or eliminating side effects, such as anomolous mobilization or disposition of calcium bone salts. Surprisingly, in addition to these benefits, the resulting treatment is more effective than if each were used alone.

The invention also encompasses treatment regimens for rheumatic conditions comprising administering to a human or animal in need of such treatment a safe and effective amount of a pharmacologically-active and pharmaceutically-acceptable organophosphate compound and a safe and effective amount of a pharmacologically-active and pharmaceutically-acceptable steroid useful in the treatment of rheumatic and inflammatory conditions.

## DETAILED DESCRIPTION OF THE INVENTION

The composition and treatment regiments of this invention employ: (1) a safe and effective amount of a pharmacologically-active and pharmaceutically-acceptable steroid useful in the treatment of rheumatic conditions; and (2) a safe and effective amount of a pharmacologically-active and pharmaceutically-acceptable organophosphate compound.

By "safe and effective amount of pharmacologically-active steroid", as used herein, is meant sufficient steroid compound to treat a rheumatic condition, such as rheumatoid arthritis, at a reasonable benefit/risk ratio attendent with any medical treatment, when used in the manner of this invention. Within the scope of sound medical judgment, the dosage of steroid will vary with the particular condition being treated, the severity of the condition, the duration of the treatment, the physical and medical characteristics of the patient, especially any predisposition to steroid complications, and the specific steroid and phosphonate compounds employed.

By "safe and effective amount of pharmacologically-active organophosphonate compound", as used herein, is meant a sufficient amount of organophosphonate compound to effectively treat the bone

erosion which accompanies rheumatic conditions, and, in addition, to minimize the steroid-induced abnormalities and inhibition of bone mineralization, at a reasonable benefit/risk ratio attendent with any medical treatment. Within the scope of sound medical judgment, the dosage of organophosphonate will vary with the particular condition being treated, the severity of the condition, the duration of the treatment, and the physical and medical characteristics of the patient, as well as the specific organophosphonates and steroids employed.

By "pharmaceutically-acceptable", as used herein, is meant that the drug compounds and other ingredients used in the present compositions and methods of treatment are suitable for use in contact with the tissues of humans and lower animals without undue toxicity, irritation, allergic response, and the like, within the scope of sound medical judgment and commensurate with a reasonable benefit/risk ratio.

The term "administration" of the compounds and compositions, as used herein, includes systemic dosage, (as by injection, especially parentally), intravenous infusion, suppositories, and oral administration thereof, as well as topical application of the compounds and compositions to the situs intended to be affected, usually the afflicted situs.

By the term "comprising", as used herein, is meant that various other compatible drugs and medicaments, as well as inert ingredients, can be concommitently employed in the compositions and processes of this invention, as long as the critical organophosphonate compounds and steroids are used in the manner herein disclosed.

By "compatible", as used herein, is meant that the components of the compositions are capable of being concommitently and/or conjointly used and comingled without interacting in a manner which would substantially decrease the efficacy of the total compositions under the conditions the invention is being employed.

By "carrier", as used herein, is meant a liquid, fluid or solid material which can optionally be used to provide finished compositions for systemic, topical or oral administration.

All percentages used herein are by weight, unless otherwise specified.

Any pharmaceutically-acceptable steroid, useful in the treatment of any rheumatic or inflammatory condition may be used in the present invention. The preparation and use of such steroid components are well known in the pharmaceutical arts. They are described in detail in Miller and Munro, Drugs, 19, 119-134 (1980), incorporated herein by reference, and by Goodman and Gilman, The Pharmacological Basis of Therapeutics, 1472-1506 (1975), also incorporated herein by reference.

As will be appreciated, a number of such steroids, both endogenous and synthetic, are effective in the treatment of rheumatic or inflammatory conditions; these include, but are not limited to, cortisone (17α, 21-Dihydroxy-4-pregnene-3, 11, 20-trione) and its derivatives [cortisone, 21 β-cyclopentyl propionate; cortisone 21-dihydrogen phosphate]; Hydrocortisone (11 β, 17, 21-Trihydroxy pregn-4-ene-3, 20-dione) and its derivatives [17-hydroxy corticosterone 21-acetate; 11 β, 17-Dihydroxy-21-(phosphonooxy) pregn-4-ene-3, 20-dione; sodium 17-hydroxycorticosterone 21-succinate; hydrocortisone 21-β,-dimethylbutyrate]; Prednisolone (11 β, 17, 21-Trihydroxypregna-1, 4-diene-3, 20-dione) and its derivatives (N, N-Diethylglycine 11 β, 17-dihydroxy-3, 20-dioxopregna-1, 4-dien-21-yl ester; 11 β, 17, 21-Trihydroxy-pregna-1, 4-diene-3, 20-dione 21-(dihydrogen phosphate) disodium salt; 11 β, 17, 21-trihydroxy pregna-1, 4-diene-3, 20-dione 21-ester with glycolic acid stearate; 21-(3-Carboxy-1-oxopropoxy)-11 β, 17-dihydroxy pregna-1, 4-diene-3, 20-dione monosodium salt; 11 β, -17-Dihydrox-21-[(3-sulfobenzoyl)oxy] pregna-1, 4-diene-3, 20-dione monosodium salt; 11 β, -17, 21-trihydroxypregna-1, 4-diene-3, 20-dione 21-(3,3-dimethylbutyrate); 11 β, 17, 21-Trihydroxypregna-1, 4-diene-3, 20-dione 21-Pivalate) as well as Prednisone (17, 21-dihydroxy pregna-1, 4-diene-3, 11, 20-trione) and its derivatives (11 β, 17, 21-Trihydroxypregna-1, 4-diene-3, 20-dione 17-valerate; 11 β, 17, 21-Trihydroxy-16-methylenepregna-1, 4-diene-3, 20-dione; N,N-Diethylglycine 11 β, 17-dihydroxy-16-methylene-3,

20-dioxopregna-1, 4-dien-21-yl ester); triameinolone and its derivatives; dexamethasone and its derivatives.

The preferred steroid of the present invention are glucocorticoids of the general formula:

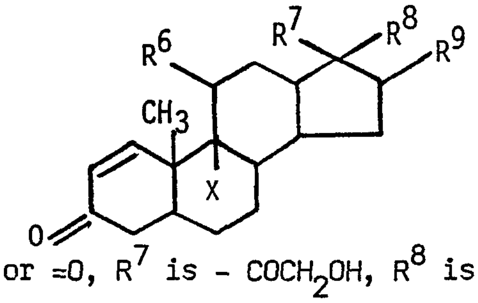

where $R^6$ is - OH or =O, $R^7$ is - $COCH_2OH$, $R^8$ is OH, and $R^9$ is H, OH or $CH_3$, X is H or F. This class of glucocorticoids includes cortisol, cortisone, predisolone, prednisone, triamcinolone, dexamethasone, pharmaceutically-acceptable salts and esters of these, and mixtures thereof. The most preferred glucocorticoids include cortisone, predisolone and prednisone. Predisolone is highly preferred.

Steroids are generally administered to patients suffering from rheumatic or inflammatory conditions on a dosing schedule which retains the therapeutic benefits of the selected steroid or steroids while minimizing the severity of potential side effects. In prescribing such a dosing regimen, the specific factors used to determine both the size and frequency of individual doses include: the severity of the underlying disorder, the anticipated duration necessary, the effective dosage that is required, the predisposition of the patient to steroid complications, the specific steroid that is selected, other modes of therapy that are concomitantly being administered, and the patient's prior exposure to steroid therapy.

Numerous schedules for such administration have been attempted with minimum side effects. However, a single alternate-day dosage schedule appears to be the most effective dosing schedule for achieving optimal therapeutic response from the patient while at the same time yielding the fewest side effects of the lowest possible severity. Because the side effects that result from steroid therapy have been shown to be related directly to the dosage and duration of administration, it is

important that the smallest possible dose be used, and that the duration of dosing also be the shortest possible period. If it is found that a short duration, such as less than two weeks, is necessary to provide the needed alleviation of symptoms, a "burst" of steroid may be used. This protocol, for example, would involve the administration on day one (of the therapy) of 100 milligrams of prednisone, 90 milligrams on day two, and reducing the dosage by 10 milligrams until the patient receives 10 milligrams of prednisone on day 10. The therapy would terminate at this point. Should it be determined that long term therapy is appropriate, a typical protocol would involve the administration of 90 milligrams of prednisone on day one, 5 milligrams on day two, 90 milligrams on day three, 5 milligrams on day four, and continuing this alternate day single dose protocol for the duration of the therapy period. Commonly, when such therapy is necessary for a very extended duration, the dosage delivered on the "high dosage" day can be reduced in small increments at intervals of approximately 5 to 7 days.

The composition of the present invention may contain from about .05 milligram of steroid to about 200 milligrams of steroid, depending upon the method of administration, the frequency of administration, and the duration of treatment. The severity of the underlying disorder is also important.

The preferred unit dosage contains from about 1 milligram per day to about 90 milligrams (per day), with about 5 milligrams (per day) to about 50 milligrams (per day) being more preferred. The most preferred unit dose is 20 milligrams (per day) of steroid.

The organophosphonate compounds employed in the present invention are characterized by the phosphonate moiety $C - (- PO_3M_2)_2$, wherein M represents H or a pharmaceutically acceptable cation or ester group. The phosphonates herein are organophosphonates, i.e., the phosphonate moiety is attached to a carbon atom by a carbon-phosphorus bond (C-P bond). The carbon atom, in turn, can be bonded to other hydrocarbyl groups, e.g. alkyl phosphonates, or to hydrogen atoms, e.g. methane phosphonates, halogen atoms, e.g. dichloromethane diphosphonates, or to mixed hydrocarbyl groups, hydrogen atoms or other

substituents, e.g. haloalkyl phosphonates. The hydrocarbyl groups can be substituted or unsubstituted alkyl (including cycloalkyl), aryl, (including heteroaryl), and the like. Substituent groups on the alkyl or aryl hydrocarbyl moiety can be, for example, additional phosphonate moieties; halogens, especially chlorine; carboxyl; esterified carboxyl; hydroxyl; amino; amido; and the like. These organophosphonates are described in U.S. Patent 3,683,080, Francis, issued August 8, 1972, incorporated herein by reference. Preferred for use herein are organophosphonates having more than one $C{-}PO_3M_2$ group; diphosphonates, especially geminal diphosphonates characterized by the group:

$$(M_2O_3P \;--\; C \;--\; PO_3M_2)$$

are most highly preferred.

Typical phosphonate compounds useful herein are selected from those having the formulae:

(I) 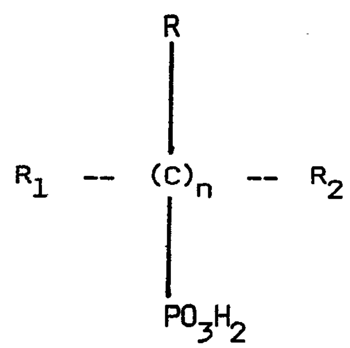 (vicinal)

(II) 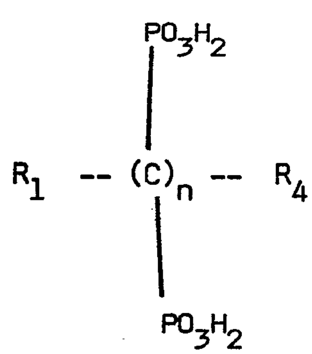 (geminal)

wherein n is an integer from 1 to about 10 and the substituent groups are H, alkyl, aryl, alkenyl, and the like. Examples of

type (I) phosphonates are those wherein R, $R_1$ and $R_2$ are each hydrogen, alkyl, $CH_2OH$, or as noted for groups $R_3$ and $R_4$. Examples of type (II) phosphonates are those wherein $R_3$ is hydrogen, an alkyl containing from 1 to about 20 carbon atoms, alkenyl containing from about 2 to about 20 carbon atoms, aryl (e.g., phenyl or naphthyl), phenylethenyl, benzyl, halogen (e.g. chlorine, bromine or fluorine), amino, substituted amino (e.g., dimethylamino, diethylamino, N-hydroxy-N-ethylamino, or acetyl-amino), $- CH_2COOH$, $- CH_2PO_3H_2$, $- CH(PO_3H_2)$ (OH) or $- CH_2CH(PO_3H_2)_2$; $R_4$ is hydrogen, lower alkyl (e.g. methyl, ethyl, propyl, or butyl), amino, benzyl, halogen, (e.g. chlorine, bromine, or fluorine) hydroxyl, $- CH_2COOH$, $- CH_2PO_3H_2$, or $- CH_2CH_2PO_3H_2$, or a pharmaceutically-acceptable salt thereof, such as alkaline metal (e.g. sodium or potassium), alkaline earth metal (e.g. calcium or magnesium), non-toxic heavy metal (e.g. stannous or indium), and ammonium or low molecular weight, substituted ammonium (e.g. mono-, di- or triethanolammonium) salts. It will also be appreciated that groups R, $R_1$ and $R_2$ and groups $R_3$ and $R_4$ can be cycloalkyl, hetercyclic or can be joined in ring structures, said rings being carbocyclic or heterocyclic.

Nonlimiting examples of phosphonates of the above type (I) include propane-1,2,3-triphosphonic acid; pentane-1,2,3,4,5-pentaphosphonic acid; butane-1,2,3,4-tetraphosphonic acid; hexane-1,2,3,4,5,6-hexaphosphonic acid; hexane-1-hydroxy-2,3,4,5,6-pentaphosphonic acid; hexane-1,6-dihydroxy,2,3,4,5-tetraphosphonic acid; heptane-1,2,3,4,5,6,7-heptaphosphonic acid; octane-1,2,3,4,5,6,7,8-octaphosphonic acid; nonane-1,2,3,4,5,6,7,8,9-nonaphosphonic acid; decane-1,2,3,4,5,6,7,8,9,10-decaphosphonic acid; and the pharmaceutically acceptable salts of these acids, e.g., sodium, potassium, calcium, magnesium, ammonium, triethanolammonium, diethanolammonium, and monoethanolammonium salts.

Among the operable phosphonates encompassed by type (II) are ethane-1-hydroxy-1, 1-diphosphonic acid; methanediphosphonic

acid; methanehydroxydiphosphonic acid; ethane-1,1,2-triphosphonic acid; propane-1,1,3,3-tetraphosphonic acid; ethane-2-phenyl-1,1-diphosphonic acid; ethane-2-naphthyl-1, 1-diphosphonic acid; methanephenyldiphosphonic acid; ethane-1-amino-1, 1-diphosphonic acid; dichloromethanediphosphonic acid; nonane-5,5-diphosphonic acid; n-pentane-1, 1,-diphosphonic acid; methanedifluorodiphosphonic acid; methanedibromodiphosphonic acid; propane-2, 2-diphosphonic acid; ethane-2-carboxy-1, 1-diphosphonic acid; propane-1-hydroxy-1,1,3-triphosphonic acid; ethane-2-hydroxy-1,1,2-triphosphonic acid; ethane-1-hydroxy-1,1,2-triphosphonic acid; propane-1,3-diphenyl-2,2-diphosphonic acid; nonane-1,1-diphosphonic acid; hexadecane-1,1-diphosphonic acid; pent-4-ene-1-hydroxy-1,1-diphosphonic acid; octadec-9-ene-1-hydroxy-1,1-diphosphonic acid; 3-phenyl-1,1-diphosphonoprop-2-ene; octane-1,1-diphosphonic acid; dodecane-1,1-diphosphonic acid; phenylaminomethane-diphosphonic acid; napththylaminomethanediphosphonic acid; N,N-dimethylaminomethanediphosphonic acid; N-(2-hydroxy-ethyl)-aminomethanediphosphonic acid; N-acetylaminomethane-diphosphonic acid; aminomethanediphosphonic acid; 3-dimethylamino-1-hydroxy-propane-1,1-diphosphonic acid; 3-diethylamino-1-hydroxy-propane-1,1-diphosphonic acid; 3-amino-1-hydroxybutane-1,1-diphosphonic acid; and the pharmaceutically acceptable salts of these acids, e.g., sodium, potassium, calcium, magnesium, stannous, indium, ammonium, triethanolammonium, diethanolammonium, and monoethanolammonium salts.

Mixtures of any of the foregoing phosphonic acids and/or salts can be used in the practice of this invention.

Dichloromethanediphosphonic acid is an especially preferred geminal diphosphonate for use herein. This compound has the molecular formula $Cl_2C(PO_3H_2)_2$, and is abbreviated $Cl_2MDP$. The dichloromethanediphosphonates, especially the sodium salts of $Cl_2MDP$, are readily prepared and are most preferred for use in the practice of this invention. Another preferred geminal diphosphonate is ethane-1-hydroxy-1,1-diphosphonic acid, abbreviated as EHDP.

Other phosphonate compounds, examples of which are given below, are well known in the art. Thus, methane-hydroxydiphosphonic acid and related compounds are described in U.S. Patent 3,422,137, Quimby, issued January 14, 1969; ethane-1,1,2-triphosphonic acid and related compounds are described in U.S. Patent 3,551,339, Quimby, issued December 29, 1970; propane-1,1,3,3-tetraphosphonic acid and related compounds are described in U.S. Patent 3,400,176, Quimby, issued September 3, 1968; pentane-2, 2-diphosphonic acid and related compounds are described in Kosolopoff, J. Amer. Chem. Soc., 75: 1500 (1953); propane-1,2,3-triphosphonic acid and salts thereof are described in U.S. Patent 3,743,688, Nicholson and Campbell, issued July 3, 1973; butane-1,2,3,4-tetraphosphonic acid and salts thereof are described in U.S. Patent 3,755,504, Nicholson and Campbell, issued August 28, 1973; the higher aliphatic vicinal polyphosphonates and salts thereof are described in U.S. Patent 3,584,035, Nicholson and Campbell, issued June 8, 1971; substituted ethane diphosphonic acids and salts and esters thereof are described in U.S. Patent 3,940,436, Kerst, issued February 24, 1976; halogenated and hydroxylated geminal diphosphonates are disclosed in U.S. Patent 3,944,599, Kerst; phosphonobutane tri- and tetra- carboxylic acid are disclosed in U.S. Patent 3,886,204, Geffers, et al.; and various amino phosphonate compounds are described in German specification 2,343,146 (March 6, 1975), German specification 2,360,711 (June 12, 1975), German specification 2,360,719 (June 6, 1975), U.S. Patent 3,962,432, Schmidt-Dunker, issued June 8, 1976, and U.S. Patent 4,054,598, Blum et al., issued October 18, 1977. The disclosures of all of the above patents and articles are incorporated herein by reference.

While any pharmaceutically-acceptable salt of the phosphonates can be used in the practice of this invention, the sodium salts are preferred. Various pharmaceutical cations such as potassium, ammonium, mono-, di- and tri-ethanolammonium, and mixtures thereof, are also suitable for use as counterions in the salts, provided caution is observed in regulating the total intake of cation species in the salt compositions. Such salts can be prepared by any suitable method involving neutralization of the

parent phosphonic acid.

The geminal diphosphonates are most preferred for use herein; preferred members of this group are of the formula:

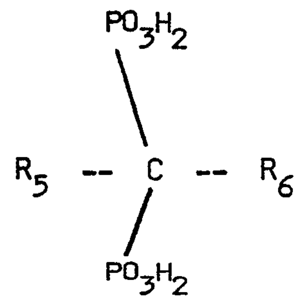

where $R_5$ and $R_6$ are H, OH, Cl, $CH_3$, $(CH_2)_3CH_3$ or $(CH_2)_2NH_2$ and pharmaceutically-acceptable salts thereof.

Ethane-1-hydroxy-1,1-diphosphonic acid is a preferred geminal diphosphonate for use herein; its preparation is disclosed in U.S. Patent 3,400,149, Quimby, issued September 3, 1968, incorporated herein by reference. This compound has the molecular formula $CH_3C(OH)(PO_3H_2)_2$ and according to nomenclature by radicals, the acid may also be named 1-hydroxyethylidene diphosphonic acid. The most readily crystallizable salt of this acid is obtained when two or three of the acid hydrogens are replaced by sodium. Preferred salts for the purpose of this invention are the trisodium hydrogen salt, the disodium dihydrogen salt, and/or mixtures thereof.

Methanediphosphonic acid and its salts are also preferred for use herein; the compounds and the methods for preparing those compounds are described in detail in U.S. Patent 3,213,030, Diehl, granted October 19, 1965 and U.S. Patent 3,251,907, Roy, granted May 17, 1966, both of which are incorporated herein by reference.

The present invention is most conveniently practiced by administering a single composition which comprises a mixture of steroid and phosphonate. In an alternate mode, the dosage regimen can consist of separate administration of the two agents; this latter approach is less convenient, but may be desirable based on the pharmacological properties of the particular compounds administered or the needs of a particular patient.

Compositions comprising the steroid and phosphonate

components can be administered parenterally in aqueous solution by subcutaneous, intradermal, intramuscular or intravenous injection.

When administered orally, the phosphonate compounds herein are only about 10% absorbed through the gut, the rest being excreted. Accordingly, oral compositions typically contain an excess of the phosphonate material over that which can be effectively used in an injectable form to account for the low absorption. Dosage forms of the present invention will, generally, contain from about 10 milligrams to about 1600 milligrams of the phosphonate compound, preferably, a geminal diphosphonate, such as dichloromethanediphosphonic acid, ethane-1-hydroxy-1,1-diphosphonic acid, methanediphosphonic acid, or a pharmaceutically-acceptable salt of these materials. Of course, the total dosage of the compositions herein will be decided by the attending physician and will be determined by such factors as the type and severity of the arthritic condition being treated, the age and weight of the patient, the medical history of the patient, and like factors well known in the medical art. In general, treatment regimens according to the present invention will call for the administration of from about 10 milligrams to about 3400 milligrams per day of the diphosphonate materials.

The preferred range for the practice of this invention is the administration of about 10 to about 2000 milligrams of diphosphonate material per day. A more preferred range is about 1000 to about 1800 milligrams per day, with about 1600 milligrams per day of phosphonate material being most preferred.

For purposes of oral administration, compositions can be formulated as capsules, tablets, granules, liquids or powders. For treatment of non-human animals, compositions are preferably incorporated in animal feeds, feed supplements or feed concentrates.

Compositions comprising the steroid and phosphonate materials can be administered, per se, or, more preferably, can be administered in combination with a solid or liquid filler, diluent or encapsulating substance as a pharmaceutical carrier, e.g., materials commonly used in a manufacture of tablets, capsules, elixirs, and the like. The composition may contain such carriers

in amounts of 0-99% by weight. Some examples of the substances which can serve as pharmaceutically-acceptable carriers include pyrogen-free water; water-alcohol mixtures; saline; sugars, such as lactose, glucose and sucrose; starches, such as corn starch and potato starch; cellulose and its derivatives, such as sodium carboxymethyl cellulose, ethyl cellulose, and cellulose acetate; powdered gums; malt; gelatin; stearic acid; calcium sulfate; vegetable oils, such as peanut oil and cottonseed oil; mineral oil; polyols, such as propylene glycol, glycerin, sorbitol, mannitol, and polyethylene glycol; agar, alginic acid; as well as other non-toxic, compatible substances used in pharmaceutical formulations. Additional components, conventionally used in pharmaceutical compositions, may also be included in the present invention at their art-established levels. Wetting agents and lubricants, such as sodium lauryl sulfate, as well as coloring agents, flavoring agents tableting agents, disclosing agents, agents to enhance or retard capsule dissolution, excipients, and preservatives, as well as other compatable pharmaceutical agents, are examples of such components.

The compositions herein can be prepared by standard formulation and tableting techniques used in the pharmaceutical industry. The following examples illustrate the present compositions and their use, but are not intended to be limiting of the scope of the invention.

## Example I

The effect of the present invention on local and systemic parameters associated with rat adjuvant arthritis was demonstrated in the following manner. One hundred and twenty-five (125) male Lewis rats weighing between 160 and 180 grams each were randomly divided into 12 groups of ten rats each, except the vehicle control group which had 15 rats. Each group was placed under a treatment regimen as described in the table below for a 46 day period.

Adjuvant arthritis was induced in groups one through twelve by a single intradermal injection of heat-killed

Mycobacterium butyricum in paraffin oil. The drug treatments were begun on the same day that the adjuvant was administered, and continued on a daily basis through the study. Drug doses were adjusted each week for changes in body weight.

Paw edema was measured weekly by liquid displacement, immersing the hind legs in a liquid reservoir to the anatomical hair line. Bone changes were evaluated radiographically and by chemical analysis at the conclusion of the study. Ectopic calcification was quantitated by use of a point-counting system with grids of one millimeter square.

The diphosphonate treatment solution for use on the animals in group 2, 3, 7-12, was prepared as follows: the disodium salt of dichloromethane diphosphonic acid was dissolved in saline, adjusted to a pH of 7.4 using sodium hydroxide, and sterilized by vacuum filtration (0.2 micron). The concentration of this solution was adjusted to a concentration of 5 mg of $Cl_2$ MDP (disodium salt) per milliliter of solution. This resulted in the administration at a dose of 0.2 ml/100 gm of body weight.

The prednisolone to be administered was suspended in 0.5% methylcellulose. The concentration of this suspension was adjusted to 0.10 milligram per milliliter so that it could be administered at a dose volume of 0.5 ml/100 gm body weight.

The disodium salt of $Cl_2$MDP was administered by subcutaneous ventral flank injections. The prednisolone was administered by gastric intubation.

| GROUP | TREATMENT |
|---|---|
| 1 | Vehicle control of 0.5% methyl cellulose plus sterile saline. |
| 2 | $Cl_2$MDP, 2mg/Kg/day in sterile saline plus 0.5% methyl cellulose. |
| 3 | $Cl_2$MDP, 5mg/Kg/day in sterile saline plus 0.5% methyl cellulose. |

| | |
|---|---|
| 4 | Prednisolone, 0.25mg/Kg/day in methyl cellulose plus sterile saline. |
| 5 | Prednisolone, 0.50mg/Kg/day in methyl cellulose plus sterile saline. |
| 6 | Prednisolone, 2.5mg/Kg/day in methyl cellulose plus sterile saline. |
| 7 | $Cl_2$MDP, 2mg/Kg/day in sterile saline plus Prednisolone, 0.25mg/Kg/day in methyl cellulose. |
| 8 | $Cl_2$MDP, 2mg/Kg/day in sterile saline plus Prednisolone, 0.5mg/Kg/day in methyl cellulose. |
| 9 | $Cl_2$MDP, 2mg/Kg/day in sterile saline plus Prednisolone, 2.5mg/Kg/day in methyl cellulose. |
| 10 | $Cl_2$MDP, 5mg/Kg/day in sterile saline plus Prednisolone, 0.25mg/Kg/day in methyl cellulose. |
| 11 | $Cl_2$MDP, 5mg/Kg/day in sterile saline plus Prednisolone, 0.5mg/Kg/day in methyl cellulose. |
| 12 | $Cl_2$MDP, 5mg/Kg/day in sterile saline plus Prednisolone, 2.5mg/Kg/day in methyl cellulose. |

At the end of the treatment period, the rats were sacrificed, necropsied, and the effects of the treatment on the rats were measured in the categories listed below.

## Table 1

Group Average Spleen Weight/Necropsy Body Weight (X 100) of Animals Receiving the Various Combinations and Dose Levels of $Cl_2$MDP and/or Prednisolone

| Dose of $Cl_2MDP$ in mg/kg | Dose of Prednisolone in mg/kg | | | |
|---|---|---|---|---|
| | None | 0.25 | 0.50 | 2.50 |
| None | 0.372 | 0.312 | 0.303 | 0.252+ |
| 2 | 0.232* | 0.244* | 0.249* | 0.210* |
| 5 | 0.240* | 0.236* | 0.215* | 0.211* |

\+ Statistically different from group receiving neither $Cl_2MDP$ nor Prednisolone.

* Statistically different from group receiving no $Cl_2MDP$ at the same level of Prednisolone.

Table 2

Group Average Ash Weight of Bone from Tibias of Animals Receiving the Various Combinations and Dose Levels of $Cl_2MDP$ and/or Prednisolone

| Dose of $Cl_2MDP$ in mg/kg | Dose of Prednisolone in mg/kg | | | |
|---|---|---|---|---|
| | None | 0.25 | 0.50 | 2.50 |
| None | 179.05 | 178.19 | 178.44 | 190.12 |
| 2 | 234.37* | 238.63* | 225.61* | 240.18* |
| 5 | 244.74* | 239.57* | 239.37* | 243.74* |

* Statistically different from group receiving no $Cl_2MDP$ at the same level of Prednisolone.

Table 3

Group Average Calcium Content of Bone from Tibias of Animals Receiving the Various Combinations and Dose Levels of $Cl_2MDP$ and/or Prednisolone

| Dose of $Cl_2MDP$ in mg/kg | Dose of Prednisolone in mg/kg | | | |
|---|---|---|---|---|
| | None | 0.25 | 0.50 | 2.50 |
| None | 19.51 | 20.96+ | 20.66+ | 20.09+ |
| 2 | 22.47* | 23.01* | 23.19* | 23.31* |
| 5 | 22.77* | 23.61* | 23.97* | 23.72* |

\+ Statistically different from group receiving neither $Cl_2MDP$ nor Prednisolone.

* Statistically different from group receiving no $Cl_2MDP$ at the same level of Prednisolone.

Table 4

Group Average Phosphorus Content of Bone from Tibias of Animals Receiving the Various Combinations and Dose Levels of

$Cl_2MDP$ and/or Prednisolone.

| Dose of $Cl_2MDP$ in mg/kg | Dose of Prednisolone in mg/kg None | 0.25 | 0.50 | 2.50 |
|---|---|---|---|---|
| None | 10.39 | 10.96+ | 10.99+ | 11.40+ |
| 2 | 11.93* | 11.82* | 11.88* | 11.96* |
| 5 | 12.28* | 11.92* | 12.15* | 12.16* |

\+ Statistically different from group receiving neither $Cl_2MDP$ nor Prednisolone.

* Statistically different from group receiving no $Cl_2MDP$ at the same level of Prednisolone.

Table 5

Average Group Bone Resorption for the Various Combinations and Dose Levels of $Cl_2MDP$ and/or Prednisolone.

| Dose of $Cl_2MDP$ in mg/kg | Dose of Prednisolone in mg/kg None | 0.25 | 0.50 | 2.50 |
|---|---|---|---|---|
| None | 2.964 | 2.400 | 2.500 | 1.300+ |
| 2 | 0.300* | 0.200* | 0.100* | 0.050* |
| 5 | 0.050* | 0.250* | 0.200* | 0.000* |

\+ Statistically different from group receiving neither $Cl_2MDP$ nor Prednisolone.

* Statistically different from group receiving no $Cl_2MDP$.

Table 6

Average Group Pathological Calcification for the Various Combinations and Dose Levels of $Cl_2MDP$ and/or Prednisolone.

| Dose of $Cl_2MDP$ in mg/kg | Dose of Prednisolone in mg/kg None | 0.25 | 0.50 | 2.50 |
|---|---|---|---|---|
| None | 32.54 | 22.85+ | 23.65+ | 14.25+ |
| 2 | 6.76* | 4.80* | 4.60* | 2.30* |
| 5 | 6.55* | 3.15* | 3.65* | 0.95* |

\+ Statistically different from group receiving neither $Cl_2MDP$ nor Prednisolone.

* Statistically different from group receiving no $Cl_2MDP$.

Table 7

Group Average Serum Level of Beta Glucuronidase of Animals Receiving the Various Combinations and Dose Levels of $Cl_2MDP$ and/or Prednisolone

| Dose of $Cl_2MDP$ in mg/kg | Dose of Prednisolone in mg/kg None | 0.25 | 0.50 | 2.50 |
|---|---|---|---|---|
| None | 2.838 | 2.698 | 2.341 | 1.912+ |
| 2 | 2.433 | 2.537 | 3.161 | 2.702 |
| 5 | 1.919 | 2.048 | 1.777 | 1.469 |

+ Statistically different from group receiving neither $Cl_2MDP$ nor prednisolone.

Table 8

Group Average Fat Free Dry Weight of Bone from Tibias of Animals Receiving the Various Combinations and Dose Levels of $Cl_2MDP$ and/or Prednisolone

| Dose of $Cl_2MDP$ in mg/kg | Dose of Prednisolone in mg/kg None | 0.25 | 0.50 | 2.50 |
|---|---|---|---|---|
| None | 326.59 | 308.96 | 311.56 | 315.58 |
| 2 | 372.48* | 379.39* | 358.93* | 379.19* |
| 5 | 382.42* | 377.55* | 377.39* | 383.33* |

* Statistically different from group receiving neither $Cl_2MDP$ nor prednisolone.

It will be appreciated that $Cl_2MDP$ treatment was significantly more effective than the placebo (saline and methyl cellulose) at inhibiting paw edema, spleen weight/body weight ratio, loss of body, weight, and bone changes associated with arthritic disease. However, prednisolone alone was only effective at inhibiting paw edema, pathological caldivication, and the calcium and phosphorus in bone. It is thought that since steroids have not been shown to have a direct effect on calcification of bone, these effects are secondary to the systemic effects of the prednisolone. See A. Blackham, Agents and Actions, 7, 145-151 (1977).

More importantly, it can be clearly seen that when the $Cl_2MDP$ and prednisolone treatments were combined, the effects on all measured parameters (except body weight) associated with arthritic disease are significantly enhanced than when either of these components is used alone. Combined treatment yields a positive interaction which one would not expect without the above example as an illustration.

Example II

Capsules having the following formulation are prepared by conventional methods.

| Ingredient | milligrams per capsule |
|---|---|
| Ethane-1-hydroxy-1,1-diphosphonic acid | 500 mg |
| Prednisolone | 100 mg |

2 capsules of the type above are administered orally once daily, or once every second day, and is effective in treating patients having rheumatoid arthritis while minimizing the bone mineralization side-effects which can accompany steroid therapy. This treatment is surprisingly more effective than either of these components administered alone at equivalent doses.

The composition of Example II may also be formulated as a tablet, using conventional tableting techniques and agents.

In the composition of Example II, the ethane-1-hydroxy-1,1-diphosphonic acid is replaced, in whole or in part, by equivalent amounts of ethane-1-hydroxy-1,1-diphosphonic acid in the sodium salt form; dichloromethanediphosphonic acid or the sodium salts thereof; methanediphosphonic acid of the sodium salts thereof; equivalent results are obtained with such replacement. The compositions are also usefully formulated using 200 milligrams or 1000 milligrams of the phosphonate compound per capsule.

In the capsules of Example II, the Prednisolone is replaced, in whole or in part, by equivalent amounts of cortisone and its derivatives; hydrocortisone and its derivatives; prednisone and its derivatives; triamcenolone and its derivatives; dexametasone and its derivatives; equivalent results are obtained with such replacement.

## Example III

Capsules having the following formulation are prepared by conventional methods.

| Ingredient | milligrams per capsule |
|---|---|
| Ethane-1-hydroxy-1,1-diphosphonic acid, disodium salt | 500 mg |
| Prednisolone | 25 mg |

2 capsules of the type above are administered orally once daily, or once every second day, and is effective in treating patients having rheumatoid arthritis while minimizing the bone mineralization side-effects which can accompany steroid therapy. This treatment is surprisingly more effective than either of these components administered alone at equivalent dosages.

The composition of Example III may also be formulated as a tablet, using conventional tableting techniques and agents, or as a liquid for oral administration, using conventional liquid carriers.

In the composition of Example III, the disodium salt of ethane-1-hydroxy-1,1-diphosphonic acid is replaced, in whole or in part, by equivalent amounts of ethane-1-hydroxy-1,1-diphosphonic acid form; dichloromethanediphosphonic acid or the sodium salts thereof; methanediphosphonic acid or the sodium salts thereof; 3-eimethylamino-1-hydroxypropane-1,1-diphosphonic acid or the sodium salts thereof; equivalent results are obtained with such replacement. The compositions are also usefully formulated using 200 milligrams or 1000 milligrams of the phosphonate compound per capsule.

In the capsules of Example III, the prednisolone is replaced, in whole or in part, by equivalent amounts of cortisone and its derivatives; hydrocortisone and its derivatives; prednisone and its derivatives; triamcenolone and its derivatives; dexamethasone and its derivatives; equivalent results are obtained with such replacement.

Example IV

A composition for intramuscular injection is prepared by blending the following ingredients.

| Ingredient | milligrams per dose |
| --- | --- |
| Dichloromethanediphosphonic acid, disodium salt | 500 mg |
| Cortisone | 100 mg |
| Pyrogen-free water | Balance |

The composition of Example IV is administered by intramuscular injection once a week to a patient having rheumatoid arthritis. Over the course of treatment, the bone erosive activity associated with both arthritic conditions and steroid therapy, decrease. The bone does not tend to demineralize. Surprisingly, in addition, the effectiveness of both components administered together is significantly greater than either component administered alone.

In the composition of Example IV, the diphosphonate material is replaced, in whole or in part, by an equivalent amount of ethane-1-hydroxy-1,1-diphosphonic acid, methanediphosphonic acid, or pharmaceutically-acceptable salts of these compounds, and equivalent results are obtained.

In the composition of Example IV, the cortisone is replaced in whole or in part, by equivalent amounts of hydrocortisone, prednisone, prednisolone, triamcenolone, dexamethasone, or derivatives thereof, and equivalent results are obtained.

The unit dosage composition of Example IV may also be formulated with variations in the diphosphonate or steroid levels, depending upon the particular dosages to be administered at a given time in the course of treatment.

Example V

A patient having rheumatoid arthritis is given an intramuscular injection containing 100 milligrams of cortisone per week, and one tablet containing 1500 milligrams of dichloromethanediphosphonic acid, disodium salt, daily. The course of treatment is continued for six (6) months. The bone erosive activity associated with the arthritic condition is found to decrease and there is no bone demineralization activity. The combination is surprisingly more effective than either component administered alone.

Example VI

A composition for topical application is prepared by blending the following ingredients.

| Ingredient | % by weight |
|---|---|
| ethane-1-hydroxy-1,1-diphosphonic acid | 5% |
| decamethyl sulfoxide | 0.5% |
| cortisone | 0.5% |
| water | balance |

The composition of Example VI is applied topically every 12 hours to a patient having rheumatoid arthritis. Over the course of treatment, the bone erosive activity associated with bone arthritic conditions and steroid therapy decrease. The bone does not tend to demineralize. Surprisingly, in addition, the effectiveness of both components administered together is significantly greater than either component administered alone.

In the compositions of Example VI, the diphosphonate material is replaced, in whole or in part, by an equivalent amount of dichloromethanediphosphonic acid, disodium salt, methanediphosphonic acid, or pharmaceutically-acceptable salts of these compounds, and equivalent results are obtained.

In the composition of Example VI, the cortisone is replaced, in whole or in part, by equivalent amounts of

hydrocortisone, prednisone, prednisolone, triamcenolone, dexamethasone, or derivatives thereof, and equivalent results are obtained.

The topical composition of Example VI may also be formulated with variations in the diphosphonate or steroid levels, depending upon the particular dosage to be applied at a given time in the course of treatment.

## Example VII

A composition for administration as a suppository is prepared by blending the following ingredients.

| Ingredient | % by weight |
|---|---|
| ethane-1-hydroxy-1,1-diphosphonic acid | 5.0% |
| cortisone | 0.5% |
| cocoa butter | Balance |

The composition of Example VII is administered by anal insertion of the suppository once a week to a patient having rheumatoid arthritis. Over the course of treatment, the bone erosive activity associated with both arthritic conditions and steroid therapy decrease. The bone does not tend to demineralize. Surprisingly, in addition, the effectiveness of both components administered together is significantly greater than either component administered alone.

In the composition of Example VII, the diphosphonate material is replaced, in whole or in part, by an equivalent amount of dichloromethanediphosphonic acid, disodium salt, methanediphosphonic acid, or pharmaceutically-acceptable salts of these compounds, and equivalent results are obtained.

In the composition of Example VII, the cortisone is replaced, in whole or in part, by equivalent amounts of hydrocortisone, prednisone, prednisolone, triamcenolone, dexamethaxone, or derivatives thereof, and equivalent results are obtained.

The suppository composition of Example VII may also be formulated with variations in the diphosphonate or steroid levels,

depending upon the particular dosage to be applied at a given time in the course of treatment.

CLAIMS

1. A pharmaceutical composition characterized in that it comprises a safe and effective amount of a pharmacologically-active, pharmacologically-acceptable organophosphonate compound and a safe and effective amount of a pharmacologically-active, pharmacologically-acceptable steroid compound.

2. A composition according to Claim 1 characterized in that the organophosphonate compound is a geminal diphosphonate.

3. A composition according to Claim 1 or 2 characterized in that the organophosphonate compound is selected from the group consisting of dichloromethanediphosphonic acid, ethane-1-hydroxy-1,1-diphosphonic acid, methanediphosphonic acid, pharmaceutically-acceptable salts of these acids, and mixtures thereof.

4. A composition according to any of Claims 1-3 characterized in that it contains from 10 milligrams to 1,600 milligrams of the organophosphonate compound and from 0.5 milligram to 200 milligrams of the steroid compound.

5. A composition according to any of Claims 1-4 characterized in that the steroid compound is a glucocorticoid steroid selected from the group consisting of cortisol, cortisone, prednisolone, prednisone, triamcenolone, dexamethasone, derivatives of these steroid compounds, and mixtures thereof.

6. A composition according to any of Claims 1-5 characterized in that the organophosphonate compound is

selected from the group consisting of ethane-1-hydroxy-1,1-diphosphonic acid, pharmaceutically-acceptable salts of this acid, and mixtures thereof.

7. A composition according to any of Claims 1-6 characterized in that the steroid compound is a glucocorticoid compound selected from the group consisting of cortisone, prednisolone, prednisone derivatives of these compounds, and mixtures thereof.